# EUROPÄISCHE PATENTANMELDUNG

(11) **EP 4 800 463 A1**
(43) Veröffentlichungstag der Anmeldung: **02.09.2026**
(21) Anmeldenummer: 25221669.2
(22) Anmeldetag: 09.12.2025
(51) Int. Cl.: G02C 13/00, A61B 3/11, H04N 13/239

(54) **VERFAHREN ZUR MOBILEN BESTIMMUNG VON AUFNAHMEDATEN FÜR BRILLENGLÄSER UND MOBILES AUFNAHMESYSTEM ZUR BESTIMMUNG VON SOLCHEN AUFNAHMEDATEN**

(30) Priorität: 11.12.2024 DE 102024137124
(71) Anmelder: Ollendorf, Hans-Joachim, 39517 Tangerhütte (DE)
(72) Erfinder: Ollendorf, Hans-Joachim, 39517 Tangerhütte (DE)
(74) Vertreter: Patentanwälte Schuster, Müller & Partner mbB

(57) **Zusammenfassung**

Die Erfindung geht aus von einem Verfahren zur mobilen Bestimmung von Aufnahmedaten für Brillengläser zur Anpassung der Brillengläser an eine Brillenfassung unter Verwendung eines mobilen computerbasierten Endgerätes mit Touchscreen-Monitor (2) und einem mindestens zwei zueinander beabstandete elektronische Kameras (5; 6) aufweisenden Stereo-Kamerasystems, das den Gesichtsbereich eines Probanden, der eine anatomisch gut angepasste Brillenfassung trägt, fotografisch erfasst.

Erfindungsgemäß erzeugen die mindestens zwei Kameras (5; 6) gleichzeitig synchrone Bilder des Probanden in der Weise, dass sie mittels des mobilen computerbasierten Endgerätes durch ein Taktsignal und ein Bildauslösesignal oder ein Taktsignal, ein Bildauslösesignal und ein Blitzauslösesignal taktsynchron ausgelöst werden.

Das erfindungsgemäße mobile Aufnahmesystem weist das o. g. mobile computerbasierte Endgerät mit Touchscreen-Monitor (2) und das o. g. Stereo-Kamerasystem auf. Erfindungsgemäß weist das mobile computerbasierte Endgerät eine Synchronisationseinheit (7) auf, die ein Taktsignal und ein Bildauslösesignal oder ein Taktsignal, ein Bildauslösesignal und ein Blitzauslösesignal taktsynchron zur Auslösung der mindestens zwei elektronischen Kameras (5; 6) bereitstellt.

## Beschreibung

Die Erfindung betrifft ein Verfahren zur mobilen Bestimmung von Aufnahmedaten für die Anpassung von Brillengläsern an eine Brillenfassung nach der Gattung des Oberbegriffs des Anspruchs 1 und ein mobiles Aufnahmesystem nach der Gattung des Oberbegriffs des Anspruchs 8.

Aufnahmedaten für die Anpassung von Brillengläsern an eine Brillenfassung werden ermittelt, um ein von einem Augenarzt oder Optiker auf der Basis einer Sehschärfeuntersuchung der Augen eines Probanden ermittelten optischen Stärke eines Brillenglases dieses Brillenglas unter Berücksichtigung der Physiognomie des Gesichtsfeldes des Probanden sowie der geometrischen Beschaffenheit der von dem Probanden ausgewählten Brillenfassung derart an diese Brillenfassung anzupassen, dass die sehkorrigierende Wirkung des Brillenglases für den Proband in Hauptblickrichtung optimal zur Geltung kommen kann.

Verfahren zur mobilen Bestimmung von Aufnahmedaten für die Anpassung von Brillengläsern an eine Brillenfassung unter Verwendung eines mobilen computerbasierten Endgerätes mit Touchscreen-Monitor sind bekannt. Beispielsweise ist ein Videozentriersystem zur Ermittlung von Zentrierdaten für Brillengläser bekannt, das aus mindestens einer Bilderfassungseinrichtung, einer Bildverarbeitungseinheit mit Computer und einer Bedieneinheit besteht, wobei alle Bestandteile in einem mobilen Gehäuse integriert sind. Die Bilderfassungseinrichtung ist auf ein Objekt im Raum mit einer definierten Entfernung ausrichtbar. Auf der Vorderseite des Gehäuses sind Mittel zur Darstellung von Sehzeichen und auf dessen Rückseite ein Bildschirm zur Wiedergabe des von der Bilderfassungseinrichtung erfassten Bildes des Objekts angeordnet. Das Vidoezentriersystem weist mindestens einen Lage- und Beschleunigungssensor auf (DE 10 2011 009 646 A1).

Bekannt ist ferner ein Handgerät zum Ausrichten einer Linse auf das Auge eines Patienten. Das Gerät umfasst eine Erfassungsvorrichtung zum Erfassen und Speichern eines Bildes eines Patienten, der eine Brille trägt, einen Prozessor zum Bestimmen der Mitte der Pupille des Patienten auf dem Bild und zum Anzeigen der Position der Linse über dem Auge des Patienten auf einem Display, wobei der optische Mittelpunkt der Linse auf die Pupille des Patienten ausgerichtet ist (US 2010/0220285 A1).

Bekannt ist auch ein Verfahren zum Messen mindestens eines geomorphometrischen Parameters einer Person, die eine Brille trägt, wobei das Verfahren ein eigenständiges Computergerät umfasst, das Folgendes umfasst: einen Bildschirm, ein Ziel, ein kompaktes Bildaufnahmesystem, das ein Mittel zum Bestimmen seiner Neigung umfasst und mit dem Bildschirm verbunden ist, und eine Verarbeitungseinheit, die es ermöglicht, das Bildaufnahmesystem zu steuern und die erhaltenen Bilder zu verarbeiten. Das Verfahren gemäß der Erfindung umfasst die folgenden Schritte:

Anpassen der Neigung des Bildaufnahmesystems, sodass es in einem akzeptablen Winkelbereich liegt; Anpassen der Höhe des Bildaufnahmesystems, sodass die Person das Ziel entlang einer im Wesentlichen horizontalen Sichtlinie sehen kann; Beobachtung des Ziels durch die Person in einer bequemen Position ohne Positionsbeschränkungen; Erfassen mindestens eines Bildes der Augen oder der Person und des Rahmens in der genannten Beobachtungsposition; Verarbeiten des mindestens einen Bildes unter Verwendung der Verarbeitungseinheit, um jeden geomorphometrischen Parameter der Person anhand der Position ihrer Pupillen und der Neigung ihrer Brille zu bestimmen; und Rücksenden des Ergebnisses der Messungen (US 2014/0240664 A1).

Bekannt ist ferner ein Verfahren zur Bestimmung der Sehparameter einer Person, das die folgenden Schritte umfasst: Aufnehmen von zwei Bildern aus zwei verschiedenen Winkeln des Gesichts der Person mit einer Kamera, wobei sich die Person in einer natürlichen Fernsichtposition befindet, Bestimmen der Neigung der Kamera relativ zum Boden zum Zeitpunkt der Aufnahme der Bilder, Ableiten der Neigung und der Richtung des Gesichts der Person daraus. Die Kamera ist bei der Aufnahme der Bilder außerhalb des fovealen Bereichs der Person angeordnet (US 2015/0339511 A1).

Bekannt sind auch ein Verfahren, eine Vorrichtung und ein Computerprogramm zur Bestimmung mindestens eines optischen Parameters eines Brillenglases, wobei bei dem Verfahren ein Bild unter Verwendung des Brillenglases aufgenommen wird und mindestens ein optischer Parameter des Brillenglases mittels Bildverarbeitung des Bildes ermittelt wird, wobei das Bild eine die Augen einschließlich der Augenpartie und/oder eine an die Augen angrenzende Gesichtspartie eines Probanden umfasst. Die Vorrichtung umfasst mindestens eine Kamera, ggf. auch eine Stereokamera, die zur Aufnahme eines Bildes unter Verwendung eines Brillenglases eingerichtet ist und das Bild wie im Verfahren beschrieben aufnimmt. Die Vorrichtung umfasst ferner eine Auswerteeinheit, die zur Bestimmung mindestens eines optischen Parameters eines Brillenglases mittels Bildverarbeitung des Bildes eingerichtet ist, sowie ggf. auch Mittel zur Bestimmung des Abstandes zwischen der Kamera und dem Auge des Nutzers. (EP 3 730 998 A1). Verfahren und Vorrichtung dienen der Bestimmung mindestens eines optischen Parameters eines Brillenglases, wozu mindestens ein Brillenglas verwendet werden muss. Damit ist es weder für den der Erstellung von Brillengläsern vorgelagerten Prozess des Einpassens der Brillengläser in eine Brillenfassung noch den Prozess der Erstellung eines Brillenglases selbst geeignet, da hierfür Aufnahmedaten benötigt werden, die durch die Physiognomie des Probanden sowie die von ihm gewählte Brillenfassung bestimmt sind.

Bekannt ist ferner ein computerimplementiertes Verfahren zur Messung mindestens eines optischen Parameters zur Anpassung eines Brillengestells an einen Träger, bei dem mit einer mobilen Einrichtung Bilder eines Trägers mit Brillengestell aus unterschiedlichen Winkeln aufgenommen werden. Aus den Bildern wird mindestens ein Umriss des Brillengestells erfasst, aus dem der mindestens eine Anpassungsparameter des Brillengestells abgeleitet wird. Die Umrissbestimmung erfolgt mittels künstlicher Intelligenz (EP 4 249 994 A1). Der Nachteil dieses Verfahrens besteht darin, dass mehrere Aufnahmen in unterschiedlichen Winkeln der Kamera zum Probanden gemacht werden müssen, wodurch das Verfahren verhältnismäßig aufwändig ist und der Proband durch die Dauer des Verfahrens einer bestimmten physischen Belastung ausgesetzt ist.

Schließlich ist ein Verfahren zur Bestimmung mindestens eines Parameters in Verbindungmit einer augenoptischen Vorrichtung bekannt, die mindestens zwei Kameras aufweist, die in der gleichen vertikalen Ebene mit horizontalem Abstand zueinander angeordnet sind. Die Bestimmung erfolgt anhand eines aufgenommenen Bildes des Gesichts der Person, die eine ausgewählte Fassung trägt. Die zweite Kamera ermöglicht es, nach der Verarbeitung mit den von der Hauptkamera erhaltenen Bildern eine dreidimensionale Darstellung der Szene zu erhalten, die auch als stereoskopisches Bild bezeichnet wird. Das Verfahren umfasst drei Schritte:
1. Erfassen mindestens eines charakteristischen Punktes mindestens eines Auges der auf dem Bild erfassten Person und Schätzung der dreidimensionalen Position des oder der erfassten charakteristischen Punkte;
2. Erkennen der getragenen Fassung und Schätzen der dreidimensionalen Position der getragenen Fassung durch Ausrichten einer dreidimensionalen Darstellung der getragenen Fassung mit der getragenen Fassung in dem erfassten Bild; und
3. Bestimmen eines oder weiterer Parameter aus der relativen Position der Augen in Bezug auf die dreidimensionale Darstellung der ausgewählten Fassung (US 11 158 082 B2).

Bei diesem Verfahren werden die Aufnahmedaten über ein Referenzbrillengestell ermittelt, was nicht nur einen Umweg bedeutet, sondern von dem Probanden ein längeres Verharren in ein und derselben Position verlangt, was für den Probanden praktisch nicht möglich ist, zumindest aber eine hohe physische Belastung bedeutet. Jede auch noch so kleine Abweichung von der Ausgangsposition des Kopfes stellt eine nicht unerhebliche Messfehlerquelle dar. Da von dem Probanden praktisch keine exakt gleichen Positionen von Referenzbrillengestell und auszumessendem Brillengestell im 3D-Raum der Kameras eingehalten werden können, sind die Messergebnisse in der Regel fehlerhaft.

Bekannt sind auch computergestützte Stereokamerasysteme, bei denen die Bildaufnahmeauslösung (Shot) durch den Bediener über eine Computerfunktion oder automatisch durch den Computer selbst ausgelöst werden, in dem über eine oder zwei Schnittstellen des Computers die Kameras einen Auslösebefehl (Shot) erhalten. Bekannt ist ebenfalls, dass die elektronischen Kameras mit eigenen Taktfunktionen, beispielsweise mit Quarztaktgeneratoren, ausgerüstet sind. Beide technische Lösungen haben den Nachteil, dass die beiden Bildaufnahmen der einzelnen Kameras nicht absolut synchron sind. So entsteht beispielsweise bei der Bildaufnahmeauslösung (dem Shot-Befehl) über einen l²C-Bus des Computers eine Zeitverzögerung von 12-15ms zwischen den einzelnen Kameras. Hinzu kommt, dass die Taktgeneratoren der einzelnen Kameras nicht synchronisiert sind und damit auch nicht zum exakt gleichen Zeitpunkt die Aufnahmen durchführen können. Dies bedeutet, dass bei der Aufnahme bewegter Objekte, beispielsweise Menschen, von beiden Kameras nicht die exakt gleiche Position aufgenommen wird und die anschließende stereoskopische Messung zu Messfehlern führen kann.

Die Aufgabe der Erfindung besteht somit in der Schaffung eines Verfahrens zur mobilen Bestimmung von Aufnahmedaten für Brillengläser zur Einpassung in eine Brillenfassung, das gegenüber dem Stand der Technik einen geringeren technischen und zeitlichen Aufwand und somit auch eine geringere physische Belastung für den Probanden bei einer hohen Genauigkeit und Zuverlässigkeit der Aufnahmedaten ermöglicht. Aufgabe der Erfindung ist es ferner, unter Verwendung herkömmlicher mobiler PC-Technik ein leicht handhabbares mobiles Aufnahmesystem zur Bestimmung von Aufnahmedaten für Brillengläser zur Anpassung an eine Brillenfassung zu schaffen, das die Messfehler des Standes der Technik vermeidet.

### Die Erfindung und ihre Vorteile

Das erfindungsgemäße Verfahren hat den Vorteil, dass es keine maßstabgebenden Hilfselemente, wie z. B. Visiermessbügel oder Referenzbrillengestelle erfordert. Es ist nur noch eine Frontaufnahme des Probanden erforderlich, wodurch sich auch die Belastung des Probanden in vertretbaren Grenzen hält.

Erfindungsgemäß wird das dadurch erreicht, dass unter Verwendung eins mobilen computerbasierten Endgerätes mit Touchscreen und einem mindestens zwei zueinander beabstandete elektronische Kameras aufweisenden Stereo-Kamerasystems der Gesichtsbereich eines Probanden, der eine anatomisch gut angepasste Brillenfassung trägt, fotografisch erfasst wird, wobei die mindestens zwei Kameras gleichzeitig synchrone Bilder des Probanden in der Weise erzeugen, dass sie mittels des mobilen computerbasierten Endgerätes durch ein Taktsignal und ein Bildauslösesignal oder ein Taktsignal, ein Bildauslösesignal und ein Blitzauslösesignal taktsynchron ausgelöst werden. Dadurch werden technisch bildgleiche Aufnahmen von dem Gesicht des Probanden erstellt.

In einer vorteilhaften Ausgestaltung der Erfindung werden die mindestens zwei von einem Minicomputer gesteuerten Einzelkameras senkrecht untereinander in Position gebracht, so dass die synchronen Bilder auf dem Touchscreen vertikal übereinander dargestellt werden.

In einer zusätzlichen vorteilhaften Ausgestaltung der Erfindung werden durch den Auslösevorgang die beiden Portraitaufnahmen in etwa mittiger Position des Bildbereichs des Touchscreen-Monitors als synchrone Standbilder auf den jeweiligen Bildhälften dargestellt.

In einer ebenfalls vorteilhaften Ausgestaltung der Erfindung werden beide Bilder in waagerechter Ausrichtung der Kameras erstellt.

In einer besonders vorteilhaften Ausgestaltung der Erfindung wird in dem Gesamtlivebild auf dem Touchscreen-Monitor eine Wasserwaage angezeigt, nach der der Bediener die optischen Achsen der mindestens zwei Kameras in Nullblickrichtung des Probanden ausrichtet. Die virtuelle Wasserwaage erleichtert es dem Bediener, das System so auszurichten, dass die optische Achse der beiden Kameras in horizontal waagerechter Position verläuft, so dass beide Portraitaufnahmen von dem Probanden in etwa in Nullblickrichtung, d. h. frei geradeaus, erstellt werden können. Durch eine manuelle Auslösung am Touchscreen oder computergesteuerte automatische Auslösung der Kameras werden auf den jeweiligen Bildhälften synchrone Standbilder als symmetrisch übereinanderliegende Portraitaufnahmen erzeugt.

In einer zusätzlichen vorteilhaften Ausgestaltung der Erfindung wird in dem Gesamtlivebild auf dem Touchscreen-Monitor eine räumliche Wasserwaage angezeigt, mittels der der Bediener die optischen Achsen der mindestens zwei Kameras zusätzlich noch in senkrechter und/oder waagerechter Position ausrichtet. Dadurch können Fehler 2. Ordnung bei der Streckenmessung ausgeglichen werden.

In einer anderweitigen vorteilhaften Ausgestaltung der Erfindung werden die erzeugten Standbilder von dem Probanden mittels Stereobildauswertung ausgewertet.

Das erfindungsgemäße mobile Aufnahmesystem zur Bestimmung von Aufnahmedaten für Brillengläser zur Anpassung der Brillengläser an eine Brillenfassung besteht aus einem mobilen computerbasierten Endgerät mit Touchscreen-Monitor und einem Stereo-Kamerasystem, das mindestens zwei zueinander beabstandete elektronischen Kameras aufweist. Das mobile computerbasierte Endgerät weist eine Synchronisationseinheit auf, die ein Taktsignal und ein Bildauslösesignal oder ein Taktsignal, ein Bildauslösesignal und ein Blitzauslösesignal taktsynchron zur Auslösung der mindestens zwei elektronischen Kameras bereitstellt.

Das erfindungsgemäße mobile Aufnahmesystem ist leicht handhabbar und ermöglicht eine hohe Genauigkeit auch in Blickrichtung der Kameras, der sog. Tiefenwerte in Richtung der z-Achse der Kamera, durch eine Stereobildauswertung. Ferner werden die durch eine Zeitverzögerung der Aufnahmen des Stereokamerasystems bedingten Messfehler durch eine in den Computer des mobilen Aufnahmesystems implementierte taktsynchrone Aufnahmetechnik vermieden.

In einer vorteilhaften Ausgestaltung der Erfindung sind die mindestens zwei zueinander beabstandeten elektronischen Kameras zum Zeitpunkt der Aufnahme in einer vertikalen Position übereinander angeordnet, wobei die optische Achse jeder Kamera bei der Aufnahme in horizontal waagerechter Richtung ausgerichtet ist. Zur Erleichterung für den Bediener die optische Achse jeder Kamera bei der Aufnahme in Nullblickrichtung des Probanden auszurichten, ist in dem Gesamtlivebild auf dem Touchscreen-Monitor eine Wasserwaage, vorzugsweise eine räumliche Wasserwaage dargestellt, mittels der der Bediener die optischen Achsen der mindestens zwei Kameras zusätzlich noch in senkrechter und/oder waagerechter Position ausrichten kann.

Weitere Vorteile und vorteilhafte Ausgestaltungen der Erfindung sind der nachfolgenden Beschreibung, den Ansprüchen und den Zeichnungen entnehmbar.

### Zeichnung

Ein bevorzugtes Ausführungsbeispiel eines mobilen Aufnahmesystems ist in den Zeichnungen dargestellt und wird im Folgenden näher erläutert. Es zeigen
- Fig. 1: eine Prinzipdarstellung eines mobilen Aufnahmesystems und
- Fig. 2: ein Blockschaltbild des mobilen Aufnahmesystems.

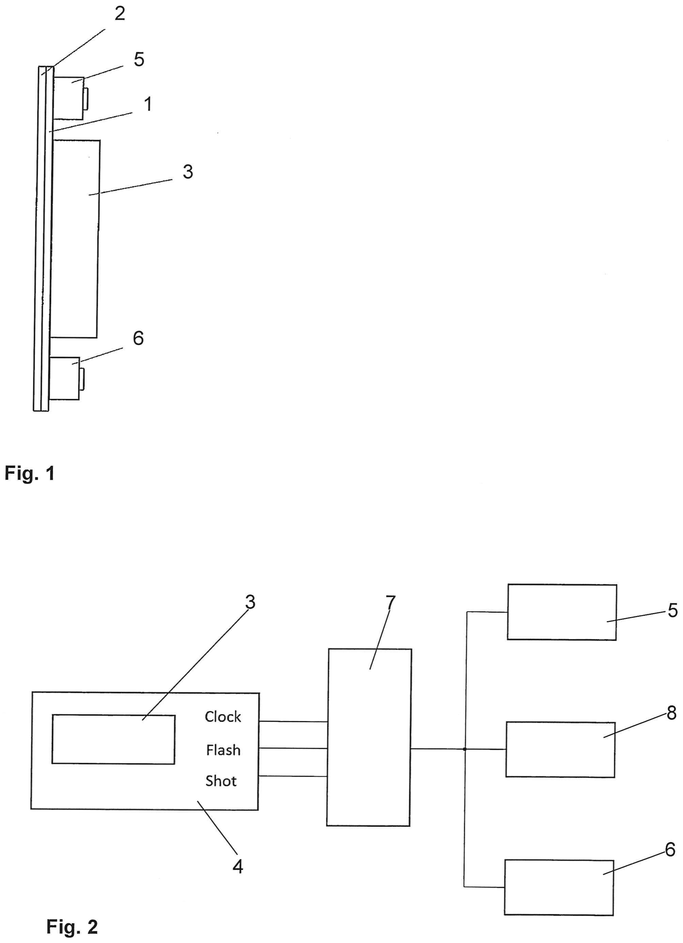

Wie aus Fig. 1 zu erkennen, weist das mobile Aufnahmesystem ein Chassis 1 auf, an deren dem Bediener zugewandten Seite ein Touchscreen 2 angeordnet ist. Auf der der aufzunehmenden Person, zugewandten Seite befinden sich ein Minicomputer 3 mit seiner System-Hauptplatine, also dem Mainboard 4, sowie eine obere Kamera 5 und eine untere Kamera 6.

Fig. 2 zeigt ein Blockschaltbild eines erfindungsgemäßen mobilen Aufnahmesystems. Das Mainboard 4 des Minicomputers 3 ist mit einer Synchronisationseinheit 7 verbunden, die die beiden Kameras 5, 6 sowie ein Blitzlicht (Flash) 8 des Aufnahmesystems ansteuert. Von dem Minicomputer 3 werden über dessen System-Hauptplatine, also dem Mainboard 4, der Synchronisationseinheit 7 ein Taktsignal (Clock), ein Bildauslösesignal (Shot) sowie, falls erforderlich, ein Blitzauslösesignal (Flash) bereitgestellt. Die Synchronisationseinheit 7 beinhaltet reine Hardwarelösungen, die verzögerungsfrei den beiden Kameras 5, 6 und ggf. dem Blitzlicht 8 zeitgleich die Auslösesignale (Shot, Flash) übermitteln. Dadurch, dass die beiden Kameras 5, 6 und ggf. das Blitzlicht 8 über das zentrale Taktsignal (Clock) taktsynchron angesteuert und ausgelöst werden, ist die Verarbeitung, d.h. die Aufnahme selbst, auch in beiden Kameras 5, 6 absolut zeitgleich (synchron). Die auf diese Weise erzeugten Aufnahmen sind Grundlage für eine fehlerfreie Bestimmung der Aufnahmedaten.

Alle hier dargestellten Merkmale können sowohl einzeln als auch in beliebiger Kombination miteinander erfindungswesentlich sein.

### Bezugszahlenliste

- 1: Chassis
- 2: Touchscreen
- 3: Computer
- 4: Mainboard
- 5: Obere Kamera
- 6: Untere Kamera
- 7: Synchronisationseinheit
- 8: Blitzlicht

## Patentansprüche

1. Verfahren zur mobilen Bestimmung von Aufnahmedaten für Brillengläser zur Anpassung der Brillengläser an eine Brillenfassung unter Verwendung eines mobilen computerbasierten Endgerätes mit Touchscreen-Monitor (2) und einem mindestens zwei zueinander beabstandete elektronische Kameras (5; 6) aufweisenden Stereo-Kamerasystems, das den Gesichtsbereich eines Probanden, der eine anatomisch gut angepasste Brillenfassung trägt, fotografisch erfasst, **dadurch gekennzeichnet,**
**dass** die mindestens zwei Kameras (5; 6) gleichzeitig synchrone Bilder des Probanden in der Weise erzeugen, dass sie mittels des mobilen computerbasierten Endgerätes durch ein Taktsignal und ein Bildauslösesignal oder ein Taktsignal, ein Bildauslösesignal und ein Blitzauslösesignal taktsynchron ausgelöst werden.

2. Verfahren nach Anspruch 1,
**dadurch gekennzeichnet,**
**dass** das mobile Endgerät so auf den Gesichtsbereich des Probanden gerichtet wird, dass sich die mindestens zwei Kameras (5; 6) in einer vertikalen Position übereinander befinden, so dass die synchronen Bilder auf dem Touchscreen-Monitor (2) vertikal übereinander dargestellt werden.

3. Verfahren nach Anspruch 1 oder 2,
**dadurch gekennzeichnet,**
**dass** durch den Auslösevorgang die beiden Portraitaufnahmen in etwa mittiger Position des Bildbereichs des Touchscreen-Monitors (2) als synchrone Standbilder auf den jeweiligen Bildhälften dargestellt werden.

4. Verfahren nach Anspruch 1, 2 oder 3,
**dadurch gekennzeichnet,**
**dass** beide Bilder in waagerechter Ausrichtung der Kameras (5; 6) erstellt werden.

5. Verfahren nach einem der Ansprüche 1 bis 4,
**dadurch gekennzeichnet,**
**dass** in dem Gesamtlivebild auf dem Touchscreen-Monitor (2) eine Wasserwaage angezeigt wird, nach der der Bediener die optischen Achsen der mindestens zwei Kameras (5; 6) in Nullblickrichtung des Probanden ausrichtet.

6. Verfahren nach Anspruch 5,
**dadurch gekennzeichnet,**
**dass** in dem Gesamtlivebild auf dem Touchscreen-Monitor (2) eine räumliche Wasserwaage angezeigt wird, nach der der Bediener die optischen Achsen der mindestens zwei Kameras (5; 6) zusätzlich noch in senkrechter oder waagerechter Position ausrichtet.

7. Verfahren nach einem der Ansprüche 1 bis 6,
**dadurch gekennzeichnet,**
**dass** die erzeugten Standbilder von dem Probanden mittels Stereobildauswertung ausgewertet werden.

8. Mobiles Aufnahmesystem zur Bestimmung von Aufnahmedaten für Brillengläser zur Anpassung der Brillengläser an eine Brillenfassung, bestehend aus einem mobilen computerbasierten Endgerät mit Touchscreen-Monitor (2) und einem mindestens zwei zueinander beabstandete elektronische Kameras (5; 6) aufweisenden Stereo-Kamerasystem,
**dadurch gekennzeichnet,**
**dass** das mobile computerbasierte Endgerät eine Synchronisationseinheit (7) aufweist, die ein Taktsignal und ein Bildauslösesignal oder ein Taktsignal, ein Bildauslösesignal und ein Blitzauslösesignal taktsynchron zur Auslösung der mindestens zwei elektronischen Kameras (5; 6) bereitstellt.

9. Mobiles Aufnahmesystem nach Anspruch 8,
**dadurch gekennzeichnet,**
**dass** die mindestens zwei zueinander beabstandeten elektronischen Kameras (5; 6) zum Zeitpunkt der Aufnahme in einer vertikalen Position übereinander angeordnet sind.

10. Mobiles Aufnahmesystem nach Anspruch 8 oder 9,
**dadurch gekennzeichnet,**
**dass** die optische Achse jeder Kamera (5; 6) bei der Aufnahme in horizontal waagerechter Richtung ausgerichtet ist.

11. Mobiles Aufnahmesystem nach Anspruch 8, 9 oder 10,
**dadurch gekennzeichnet,**
**dass** in dem Gesamtlivebild auf dem Touchscreen-Monitor (2) eine Wasserwaage dargestellt ist.

12. Mobiles Aufnahmesystem nach einem der Ansprüche 8 bis 11,
**dadurch gekennzeichnet,**
**dass** mittig zwischen den beiden Kameras (5; 6) eine Blickmarke angeordnet ist.
